# EUROPEAN PATENT SPECIFICATION

(11) **EP 1 898 889 B1**
(45) Date of publication and mention of the grant of the patent: **07.10.2009**
(21) Application number: 06809049.7
(22) Date of filing: 21.06.2006
(51) Int. Cl.: A61K 9/48, A61K 8/11, B01J 13/04, A23L 1/00

(54) **GELLAN SEAMLESS BREAKABLE CAPSULE AND PROCESS FOR MANUFACTURING THEREOF**
NAHTLOSE BRECHBARE GELLANKAPSEL UND HERSTELLUNGSVERFAHREN DAFÜR
CAPSULE RUPTURABLE DE GÉLLANE SANS SOUDURE ET SON PROCÉDÉ DE FABRICATION

(30) Priority: 21.06.2005 WO PCT/EP2005/008502; 05.08.2005 WO PCT/EP2005/009226
(43) Date of publication of application: 19.03.2008
(73) Proprietor: V. Mane Fils, 06620 Bar sur Loup (FR)
(72) Inventor: HARTMANN, Didier, F-06150 Cannes La Bocca (FR); HANNETEL, Jean-Michel, F-06130 Grasse (FR); COURSIERES, Nathalie, F-06530 St Cezaire sur Siagne (FR); MANE, Jean, F-06130 Grasse (FR)
(74) Representative: Cabinet Plasseraud
(86) International application number: PCT/IB2006/002905
(87) International publication number: WO 2007/012981

(56) References cited:
- EP-A- 0 513 603
- EP-A- 0 622 408
- EP-A- 1 072 633
- DE-A1- 19 926 714
- FR-A- 2 757 173
- US-A- 5 456 937
- DATABASE WPI Week 199903 Derwent Publications Ltd., London, GB; AN 1999-029026 XP002422170 & JP 10 291928 A (FUJI CAPSULE KK) 4 November 1998 (1998-11-04) cited in the application

## Description

The present invention relates to a breakable capsule having a fluid core and a solid or fluid breakable shell.

In this invention, the term "capsule" means a spherical or substantially spherical delivery system of a substance, said substance being hereinafter referred to as "the core", and said substance being encapsulated into a shell, the shell being breakable and releasing the core when broken or ruptured. The term fluid means flowing as opposed to being in a solid state. According to the invention, the term fluid includes finely divided solids, such as a powder, and also gel, or any physical state of a product wherein said product changes shape or direction uniformly, in response to an external force imposed upon it. According to the invention, fluid preferably refers to a flowable or gellified product.

The term "breakable capsule" refers to a capsule as hereabove defined, wherein the shell can be ruptured by means of a pressure, which results in the release of the core. According to an embodiment, the capsule of the invention may be specifically designed to be incorporated into a fluid medium such as for example a gel, a pasty or a liquid medium containing water; in this embodiment, the capsules may be suspended or mixed by any suitable means in order to bring an visual effect of homogeneous dispersion of the capsules in the medium; advantageously, the shell and/or the core of the capsule is coloured. According to another embodiment, the capsule of the invention is dispersed into a solid or fluid medium, such as for example a powder; advantageously, the shell and/or the core of the capsule is coloured.

Such capsules are useful for numerous applications, such as in oral care application (toothpaste, mouthwash, gums...), in food applications such as confectionary, dairy, bakery, savory, in neutraceutical applications or in pharmaceutical or in personal care products such as cosmetic products and the like.

In the present patent application, the term "capsule" will be used to designate any size of capsules, including macrocapsules and microcapsules and preferably capsule which larger diameter is from 0.5 mm up to 8 mm, preferably 1 to 5 mm; more preferably 1.2 to 3 mm.

It is of particular interest to obtain seamless capsules, as the breakability of a welded capsule (also designated in the prior art as softgel or hard capsule) may be influenced by the easy or unwanted rupture of the weld.

Fuji patent application JP10291928 describes a capsule obtained through a co-extrusion process, wherein the external liquid phase comprises gellan and calcium salts. Gellan gum, first discovered in 1978, is produced by the microorganism Sphingomonas *elodea.*

The Applicant has found that the production of gellan capsule through the Fuji process was not satisfactory and resulted in poor quality capsules and in processing difficulties, because the gellan was actually gelling during the co-extrusion, and it was not possible to obtain spherical and homogeneous breakable capsules.

For this reason, the Applicant tried to improve the Fuji process and found that the drawbacks of the prior art process may be due to the presence of calcium gale, and more generally to divalent metal salts in gellan during the co-extrusion step. Thus, the Applicant carried out a process wherein the co-extruaion liquid phase containing gellan was performed in absence of calcium salts, and observed that, surprisingly, the resulting capsules had the required spherical or substantially spherical shape and homogeneous sizes. However, the capsules thus obtained could not' be used an such, because the shell was too soft and the resulting capsules were not breakable capsules; the Applicant found a solution to this subsequent technical problem by contacting the capsules with divalent metal ions, preferably calcium or magnesium ions, or by using organic acid solution, once the co-extrusion process is finished, and this finally lead to satisfactory breakable capsule.

Thus, this invention relates to a process for manufacturing seamless breakable capsules and to new seamless breakable capsules as defined in claim 1.

The process of the invention comprises a step (A) of co-extrusion of an external and hydrophilic liquid phase and an internal and lipophilic liquid phase, in order to form a capsule having a core comprising the internal and lipophilic phase and a shell comprising the external and hydrophilic phase; and a step (B) of washing and immersing the capsules into an aqueous solution preferably containing a curing agent, the curing agent being one of the means for making the shell breakable as required for the intended use; optionally a step (C) of drying the obtained capsules or optionally a step (D) of suspending the capsules into an fluid medium.

The co-extrusion process comprises three main stages: compound drop formation, shell solidification and capsule collection. The compound drop is a sphere of the liquid fill phase inside the shell phase. The liquid fill phase is hereinafter referred to as "the core". The shell phase is hereinafter referred to as "the shell".

According to the invention, the external liquid phase includes a gelling agent comprising gellan gum, alone or in combination with at least one suitable gelling agent, a filler, and a metal sequestering agent, the liquid preferably being aqueous, more preferably the liquid is water, preferably desionized or osmozed water.

By "gelling agent" in the meaning of this invention, it is referred to an agent able to convert an aqueous phase from a flowable or fluid liquid to a solid or a gel.

By "sequestering agent" in the meaning of this invention it is referred to any agent complexing, chelating or sequestering bivalent ions such as calcium or magnesium ions.

The term "substantially", when referring to a number or value, means + or - 10 % of the value; when referring to a sphere, it means a distorted sphere which larger diameter is + or - 10 % of the diameter of the expected sphere.

The term "wet capsule" in the_ meaning of this invention, refers to a capsule which shell includes a positive amount of water. The term wet capsule is used for the calculation of percentages of ingredients in the final product or shell, as opposed to the calculation based on the dry weight of said final product or shell.

The breakable capsule according to the invention preferably" has a crush strength from 0.01 to 5 kp, preferably from 0.1 to 2.5 kp, edge values being included. The crush strength of the capsule is measured by continuously applying a load vertically onto one particle until rupture. The crush strength of the capsules in the present invention is measured by using a texturometer TA.XT plus from Micro Stable System in compression mode or a LLOYD - CHATILLON Digital Force Gauge, Model DFIS 50, having a capacity of 25Kg, a resolution of 0.02 Kg, and an accuracy of +/- 0,15 %. The force gauge is attached to a stand; the capsule is positioned in the middle of a plate that is moved up with a manual thread screw device. Pressure is then applied manually and the gauge records the maximum force applied at the very moment of the rupture of the capsule, (measured in Kg or in Lb). Rupture of the capsule results in the release of the core.

Gellan gum is a hydrocolloid which, according to the invention, can be used as the sole gelling agent of the external liquid phase, or in combination with at least one ohter gelling agent. Other suitable gelling agents may be alginates, agar, carragheenan, pectines, xanthan gum, Arabic gum, tara gum, ghatti gum, karaya gum, dextran, curdlan, welan gum, rhamsan gum or modified starches. Suitable gellan gums are for example, but not limited to deacylated gellan gum. Kelcogel^{®} can be mentioned as a suitable gellan gum.

The amount of gelling agent present in the shell is 4 to 95%, preferably 5 to 75%, even more preferably is 10 to 50%, mores preferably 12 to 40 % by weight of the total dry weight of the shell.

When used in combination with at least another gelling agent, the weight ratio between gellan gum and the other gelling agent (a) is from 80/20 to 20/80, preferably 75/25 to 25/75, and even more preferably from 60/40 to 50/50.

Preferably, the weight ratio of gelling agent / dried shell is greater than 10%, preferably greater than 12, more preferably greater than 15%.

The filler is any suitable material that can increase the percentage of dry material in the external liquid phase or bring filming properties. Increasing the dry material amount in a shell results in solidifying the shell, and in making it physically more resistant or impermeable. Preferably, the filler is selected from the group comprising starch derivatives such as dextrin, maltodextrin, polyol, cyclodextrin (alpha, beta or gamma), or cellulose derivatives such as hydroxypropylmethylcellulose (HPMC), hydroxypropylcellulose (HPC), methylcellulose (MC), carboxymethylcellulose (CMC), polyethylene glycol derivative, polyvinyl alcohol, polyole or mixture thereof.

The amount of filler in the shell is from 50 to 95% by weight on the total dry weight of the shell.

Using a divalent metal sequestering or complexing agent allows trapping the divalent metal ions which are possibly present in the components of the liquid phase including water and which have a gelling effect on gellan. Thus, the use of a divalent metal sequestering agent, preferably of a calcium ion sequestering agent, allows the gellan to be co-extruded without undesirable or uncontrollable gelling during the co-extrusion.

The amount of sequestering agent is at moat 2%, preferably at most 1% and even more preferably at moat 0.5% by weight of the total dry weight of the ahel.3.

Preferably, the water used for the external phase is deionized water and/or osmozed water; using processing water remains possible but needs adjusting the amount of divalent metal sequestering agent.

The sequestering agent is a metal salt, selected from the group comprising trisodium citrate, trisodium phosphate, tetrasodium pyrophosphate, sodium hexametaphosphate and mixtures thereof.

The hydrophilic external liquid phase may further comprise at least one plasticize, which may be at least one of glycerol, sorbitol, maltitol, triacetine or polyethylene glycol type product, or a polyalcohol with plasticizing or humectant properties. Advantageously, the hydrophilic external liquid phase further comprises at least one coloring agent or pigment; according to a first embodiment, the colouring agent or the pigment is in a form of a powder or a suspension stable in an aqueous medium. According to another embodiment of the invention, the liquid phase may include perfumes, aromas, fragrances or any adoring agent.

According to one embodiment of the invention, the co-extrusion step (A) of the process can be performed at a temperature being from room temperature to 100°C. Advantageously, it is performed at room temperature, which means between 18 and 30°C, preferably 20-25 °C under atmospheric pressure.

The co-extrusion step is a synchronous extrusion of two liquids: 1 the external and hydrophilic liquid phase, and the internal and lipophilic liquid phase which can be performed using an apparatus and a process as described in EP 513603, the disclosure of which is herein incorporated by reference.

According to an embodiment of the invention, after the co-extrusion step (A), the solidification steep is performed by keeping cold the capsules in order to ensure correct gelling of the shell, for example by contacting them with a cold bath. The cold bath may preferably be cold oil or cold emulsion. Cold means any temperature below 18"°C, preferably the temperature is from 2 to 10 °C, more preferably 4 to 6 °C.

According to an embodiment of the invention, the capsule may then be centrifuged in order to remove the surplus oil, and/or washed with organic solvent (such as acetone, ethyl acetate, ethanol, petroleum ether, etc.) also to remove the surplus oil, and optionally dried in a air flow at controlled temperature and humidity. The relative humidity of the drying air is 20% to 60%, preferably 30 to 50%; the temperature of the drying air is or .15 to 60 °C, preferably 35 to 45 °C.

The capsules are immersed into an aqueous solution or an emulsion containing a curing agent which comprises a divalent salt and optionally an acid. The effect of the immersion step is to wash out the oil remaining at the periphery of the capsules, and to gradually strengthen the shell, notably through dehydration and osmotic equilibrium.

According to one embodiment of the invention, after immersion, the capsules are dried in the same conditions as mentioned above. According to another embodiment of the invention, after immersion, the capsules are not dried.

The curing agent comprises calcium ions.

The aqueous solution or emulsion containing the curing agent is preferably a divalent metal salt solution, containing calcium salts, preferably, calcium dichloride, calcium carbonate, calcium sulfate or dicalcium phosphate. This solution may be the aqueous phase of an oil-inwurater emulsion, This solution can be at a temperature comprised between 2°C and room temperature. Advantageously, the aqueous solution containing the curing agent is maintained under acid conditions of pH, and preferably at a pH logo than 5, more preferably from 2 to 4. According to a preferred embodiment of the invention, the aqueous solution, or emulsion containing a curling agent is a calcium chloride solution having a pH of 3 to 4.

The aqueous solution containing the curing agent can also contain preservatives or bactericides such us benzoate, parabens, diols, cetylpyridinium chloride, diazolidinyl urea or any preservatives used for food, pharmaceutical or cosmetic products.

According to one embodiment of the invention, the process comprises the steps of co-extruding the above mentioned external and internal liquid phases, optionally solidifying and/or gelling the surface of the shell by keeping the capsule under cold conditions, as explained herein above, optionally centrifugating, optionally washing the so-obtained capsules with an organic solvent, immersing the resulting capsules into an aqueous solution containing a curing agent, and optionally drying the capsules.

According to one embodiment of the invention, the solidifying/gelling/curing steps can be gathered into a single step, for example by dipping the capsules into a bath, under cold conditions, containing the calcium, more preferably, calcium dichloride, calcium sulfate or dicalcium phosphate. This bath may be an oil-in-water emulsion.

The capsules manufactured through the profess according to the invention are substantially or perfectly spherical and very homogeneous in size.

This invention also relates to breakable capsule which are preferably seamless capsules susceptible to be obtained through the process according to the invention.

The capsules of the invention comprises a core and a shell, and said shell includes a gelling agent comprising gellan gum alone or in combination with another gelling agent, a filler, and a divalent metal sequestering agent.

Preferably the gelling agent of the shell is a combination of gellan and of at least one other gelling agent selected from the group consisting of gelatin and hydrocolloids such as agar, carragheenan, pectins, xanthan gum, alginate, tara gum, arabic gum, ghatti gum, caroub gum, cellulose gum, dextran, curdlan, welan gum, rhamsan gum or modified starches.

According to a preferred embodiment of the invention the filler and the sequestering agent, are as described hereinabove.

According to another embodiment, the shell further comprises a plasticizer as described hereinabove.

The amount of plasticizer ranges from 0.1% to 30% by weight, preferably from 2% to 15% by weight, and even more preferably from 3 to 10% by weight of the total dry weight of the shell.

According to the intended use of said capsules, the shell may contain other additives such as perfumes, aromas, or any flavoring agent.

According to the intended use of said capsule, the shell may, comprise coloring agent such as pigments, titanium dioxide, iron oxides, carbon black, or any type of food, oral care, cosmetic or pharmaceutical pigment such as Covasorb colors distributed by LCW.

The shell of a breakable capsule according to the invention represents by-weight 8 to 50% of the total weight of said capsule, preferably 10 to 40%, more preferably 20 to 30%.

The amount of water present in the shell is of 1 to 60 %, preferably 5 to 40 % the capsule remaining breakable even at the higher percentages.

According to a preferred embodiment, the breakable capsule according to the invention has a crush strength of from 0.01 to 5, preferably from 0.01 to 2.5 kp.

Advantageously, the shell thickness of the capsule is 10-500 microns, preferably 30-150 microns, more preferably 50-60 microns. The ratio diameter of the capsule/thickness of the shell is in the range of 1 to 100, preferably 5 to 30.

The core of the capsule is preferentially composed of a mixture of materials or products which are lipophilic or partially soluble in ethanol, or of molecules formulated as oil/water/oil emulsions.

The core of a breakable capsule according to the invention represents by weight 50 to 92% of the total weight of said capsule, preferably 60 to 90%, more preferably 70 to 80%.

The core of the capsule may be composed of one or more lipophilic solvents conventionally used in the food, pharmaceutical or cosmetic industries. In a preferred embodiment, these lipophilic solvents may be triglycerides, especially medium chain triglycerides, and in particular triglycerides of caprylic and capric acid, or mixtures of triglycerides such as vegetable oil, hydrogenated oil, coconut oil, palm oil, olive oil, sunflower oil, corn oil, linseed oil, cottonseed oil, groundnut oil, grape seed oil, wheat germ oil, fish oil, beet fat, mineral oils and silicone oils. The amount of lipophilic solvent in the core of a capsule according to the invention is of the order of 0.01 to 90%, preferentially 25 to 75%, of the total weight of the capsule.

The core may also comprise one or more aromatic or fragrance molecules as conventionally used in the formulation of flavoring or fragrance compositions. Mention will in particular be made of aromatic, terpenic and/or sesquiterpenic hydrocarbons, and more particularly essential oils, alcohols, aldehydes, phenols, carboxylic acids in their various forms, aromatic acetals and ethers, nitrogenous heterocycles, ketones, sulfides, disulfides and mercaptans which may be aromatic or non aromatic. It may also comprise one or more molecules or extracts for cosmetic use.

The core may also comprise one or more fillers as used in aromatic emulsions. Mention will be made of dammar gum, wood resins of the ester gum type, sucrose acetate isobutyrate (SAIB) or brominated vegetable oils. The function of these weighting agents is to adjust the density of the liquid core.

The core may also comprise one or more sweeteners, which may be provided in the form of a solution or suspension in ethanol. Examples of suitable sweeteners may be, but is not limited to, aspartame, saccharine, NHDC, sucralose, acesulfame, neotame, thaumatin, steviosides, etc.

The core may also comprise one or more "sensate" aromatic agents, which provide either a freshening effect or a hot effect in the mouth. Suitable freshening agents may be, but are not limited to, menthyl succinate and derivatives thereof, in particular Physcool^{®} marketed by the Applicant. A suitable hot effect agent may be, but is not limited to, vanillyl ethyl ether.

The flavoring agents that can be solubilized in the solvent of the core of the capsule include, but are not limited to, natural or synthetic aromas and/or fragrances. Examples of suitable fragrances are fruity, confectionery, floral, sweet, woody fragrances. Examples of suitable aromas are vanilla, coffee, chocolate, cinnamon, mint. The core may also comprise a lipophilic color such as fake colors but also natural colors such as paprika oleoresin, turmeric oleoresin, carotenes, chlorophyllin, or any other suitable natural coloring product. The core may also include lipophilic active agents, such as vitamins, more preferably vitamine B; fatty acids, preferably omega 3 and natural extracts of plants.

The capsules according to the invention can be included in various products, such as food products, oral care products, nutraceutical products, pharmaceutical products, cleaning products and cosmetic products. The invention thus relates to a food product including breakable capsules according to the invention; an oral care product including breakable capsules according to the invention, preferably a toothpaste including breakable capsules according to the invention; a pharmaceutical product including breakable capsules according to the invention; a fragrance including breakable capsules according to the invention.

The capsules of the invention may be within a slurry, in suspension in a gel, preferably carried out with a gel forming agent such as xanthan gum, gellan gum, CMC or Carbopol, araboxymethyl cellulose, or any polymer commonly used as suspending agent and optionally comprising preservatives and stabilizers.

The total weight of the capsule of the invention depends on its diameter and on the amount of core filling the shell. According to an embodiment of the invention, the total weight of the capsule is within the range of 0.1 to 50 mg, preferably 0.2 to 20 mg, more preferably 0.5 to 10 mg.

The invention is hereunder illustrated by the following examples, which should not be considered as limiting the scope of the invention.

### EXAMPLES

### Example 1

Menthol Capsules (referred as 3039/A1) are prepared by co-extruding an outer liquid phase and an internal liquid phase presenting the following compositions:

| **Outer liquid phase** Dry matter: 15,0% | **%/total weight** | **% / dry matter** | **% wet capsule** |
|---|---|---|---|
| gellan | 2.000% | 13.33 | 1.482 |
| Sorbitol | 1.000% | 6.67 | 0.741 |
| Dextrin Cristal Tex 648 | 11.400% | 76.00 | 8.445 |
| Sodium citrate | 0.200% | 1.33 | 0.148 |
| Citric acid | 0.1% | 0.67 | 0.074 |
| unipure blue pigment CI77007 | 0.300% | 2.00 | 0.222 |
| Deionized water | 85.000% | | 62.968 |
| | 100.000% | 100 | |
| | | | |
| **Internal liquid phase** | % | % | |
| Ethanol | 5.0000% | 5% | |
| Miglyol 812S | 81.5000% | 81.5% | 22.245% |
| Menthol codex | 13.5000% | 13.5% | 3.685% |
| | 100.0000% | 100.00% | 100% |

The obtained capsules are separated into two batches referred as Ala and Alb. Capsules from each batch are cooled at 4°C for 1h, washed with desionised water and then immersed in a bath containing an aqueous solution of calcium chloride (0.1% for Ala and 1% for Alb) at pH=3.5 at T=20°C during 15 minutes.

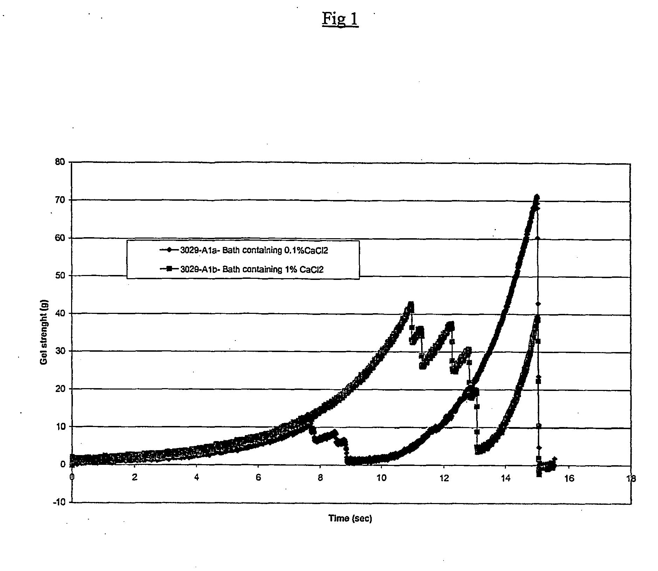

Wet capsule crush strength (gel strength) is then measured for both capsules Ala and Alb using a texturometer TA.XT plus from Micro Stable System to compare influence of concentration of calcium (the results are presented on Figure 1).

Wet capsule strength is higher using 1% CaCl2 solution than using 0.1% CaCl2 solution.

After drying, crush strength of the capsules is measured using a texturometer in compression mode.

| | 3039/A1a | 3039/A1b |
|---|---|---|
| Crush strength (dry capsules) | 184g | 186.6g |

The obtained capsules present the following physical characteristics:
diameter: 2mm,
thickness of the shell: 0.096mm,
total weight: 4mg,
weight of the core: 2.8mg (70%),
weight of the shell: 1.2mg (300).

Such capsules are then placed into a clear toothgel and bring nice visual effect of spherical blue capsules liberating menthol when broken.

### Example 2

Cinnamon Capsules (referenced as 4053/F1) are prepared by co-extruding an outer liquid phase and an internal liquid phase presenting the following compositions:

| **Outer liquid phase** Dry matter: 13.0% | **%/total weight** | **% / dry matter** |
|---|---|---|
| gellan | 2.000% | 15.38% |
| Sorbitol | 1.900% | 14.62% |
| Dextrin Cristal Tex 648 | 8.500% | 65.38% |
| Sodium citrate | 0.200% | 1.54% |
| Calcium citrate | 0.100% | 0.77% |
| Titanium dioxide | 0.300% | 2.31% |
| Osmosed water | 87.000% | 100% |
| | 100.000% | |
| **Internal liquid phase** | **%/total weight** | **% without ethanol** |
| Ethanol | 5,0000% | |
| Miglyol 812S | 58.9000% | 85,79% |
| Cinnamon | 19.60000 | 14,21% |
| Physcool | 10.0000% | 10.53% |
| N-ethyl-p-menthane-3-carboxamide commercialy available as WS3 | 6.5000% | 6.84% |
| **Total** | **100,0000%** | **100,00%** |

The obtained capsules are cooled at 4°C for 1h, washed with deionised water and then immersed in a bath containing an aqueous solution containing 1.25% of calcium chloride at pH=3 at T=20°C during 30 minutes.

The obtained capsules present the following physical characteristics:
- diameter:: 1.2mm,
- thickness of the shell :: 0.053mm,
- total weight :: 0.87mg.,
- weight of the core:: 0.62mg (71.980),
- weight of the shell :: 0.24mg (28.02%),

Capsules are then incorporated into a toothpaste base containing mint flavour and cinnamon capsules 4053/F1 at a 0.2% use level. During brushing, cinnamon flavour is clearly identified showing good breakability of the capsules.

### Example 3

Orange capsules (referred as 5053/Cl) are prepared by coextruding an outer liquid phase and an internal liquid phase presenting the following compositions:

| **Outer liquid phase** Dry matter: 15.0% | **%/total weight** | **% / wet capsule** |
|---|---|---|
| gellan | 2.000% | 0.950 |
| Sorbitol | 1.000% | 0.45% |
| Dextrin Cristal Tex. 648 | 11.4% | 5.36% |
| Sodium citrate | 0.200% | 0.01% |
| water | 84.5% | 40% |
| | 100.000% | |
| **Internal liquid phase** | **%** | **%** |
| Orange flavour | 19.905% | 5.47% |
| Coconut oil | 80% | 47.7% |
| Paprika color | 0.095% | 0.06% |
| **Total** | **100,0000%** | **100,00%** |
| | | |
| | | |

Wet capsule crush strength (gel strength) is then measured using a texturometer TA.XT plus from Micro Stable System. Crushstrength value obtained is 15g and these capsules are easily broken under the teeth.

The obtained capsules present the following physical characteristics:
- Diameter :: 2.5 mm
- Thickness of the shell:: 0.32 mm
- Total weight:: 8.2 mg

Capsules are then placed into a suspension of xanthan gum to be applied to beverage application. Capsules can be swallowed or broken under the teeth to liberate the flavour into the mouth.

### Example 4

Menthol capsules (referred as 5025/B1) are prepared by coextruding an outer liquid phase and an internal liquid phase presenting the following composition:

| | % dry capsule | | % wet capsule untreated | | % wet capsule treated with acid as calcium, releasing agent | |
|---|---|---|---|---|---|---|
| **Outer liquid phase** | % shell | %/total weight | % shell | %/total weight | % shell | %/total weight |
| gellan | 13.333% | 3.6% | 2% | 1.423% | 2% | 1.423% |
| Sorbitol | 6.667% | 1.8% | 1% | 0.712% | 1% | 0.712% |
| Dextrin Cristal Tex 648 | 76% | 20.52% | 11.4% | 8.111% | 11.4% | 8.111% |
| Sodium citrate | 1.333% | 0.360% | 0.2% | 0.142% | 0.2% | 0.142% |
| Citric acid | 0.667% | 0.180% | 0.1% | 0.071% | 0.1% | 0.071% |
| Unipure blue pigment CI77007 | 2% | 0.54% | 0.3% | 0.213% | 0.3% | 0.213% |
| water | 0% | 0% | 85% | 60.480% | 85% | 60.480% |
| | | | | | | |
| **Internal liquid phase** | | | | | | |
| ethanol | 0% | 0% | 0% | 0% | 0% | 0% |
| Miglyol 812S | 47.368% | 34.579% | 47.368% | 13.664% | 47.368% | 13.664% |
| Menthol codex | 52.632% | 38.421% | 52.632% | | 52.632% | 15.183% |
| **Total** | 100% | 73% | 100% | 28.8% | 100% | 28.8% |
| | | | | | | |
| **Total** | | 100% | | 100% | | 100% |
| | | | | | | |
| **Crush strength** | 94.31 g | | 5.09 g | | 15.09 g | |

The treatment of wet capsules with an acid as calcium remleasing agent allow the enhancing of the crush strength of the capsules.

## Claims

1. A seamless breakable capsule comprising a core and a shell, wherein the shell includes a gelling agent comprising gellan gum alone or in Kombination with another gelling agent,
**characterized in that** the_shell further includes a filler, and a divalent metal sequestering agent selected from the groupe consisting of trisodium citrate, trisodium phosphate tetrasodium pyrophosphate, sodium hexametaphosphate and mixtures thereof, wherein the amout of the filler in the shell is from 50 to 95% by weight on the total dry weight of the shell, the amount of the sequestering agent is at most 2% by weight of the total dry weight of the shell.

2. The seamless breakable capsule according to claim 1, wherein the larger diameter of the capsule is 0.5 mm to 8 mm.

3. The seamless breakable capsule according to claim 1 or 2, wherein the gelling agent is a combination of gellan and one gelling agent selected from the group consisting of gelatin, agar, carrageenan, pectins, xanthan gum, cellulose gum, alginate, dextran, curdlan, welan gum, rhamsan gum or modified starches.

4. The seamless breakable capsule According to claim 1, wherein the gelling agent is gellan gum alone.

5. The seamless breakable capsule according to claim 1, wherein, when used in combination with at least another gelling agent, the weight ratio between gellan gum and the other gelling agent(s) is from 80/20 to 20/80, preferably 75/25 to 25/75, and even more preferably from 60/40 to 50/50.

6. The seamless breakable capsule according to anyone of claims 1 to 5, wherein the filler is a starch derivative such as dextrin, maltodextrin, cyclodextrin and/or a cellulose derivative such as hydroxypropylinethylcellulose (HPMC), hydroxypropylcellulose (HPC), methylcellulose (MC), polyvinyl alcohols, polyols or mixtures thereof.

7. The seamless breakable capsule According to anyone of claims 1 to 6, wherein the shell further comprises an acid salt selected from the group comprising such as citrate, glucuronate, adipate, fumarate, gluconate and salt of glucono-delta-lactone, and mixtures thereof.

8. The seamless breakable capsule according to anyone of claims 1 to 7, having an crush strength from 0.01 to 5 kp.

9. The seamless breakable capsule according to anyone of claims 1 to 8, wherein the amount of plasticizer ranges from 0.1 to 30% by weight, preferably from 2% to 15% by weight and even more preferably from 3 to 10% by weight of the total dry weight of the shell.

10. The seamless breakable capsule according to anyone of claims 1 to 9, having an crush strength from 0.01. to 5 kp.

11. A process for manufacturing a seamless breakable capsule according to anyone of claims 1 to 10, comprising
- co-extruding an external and hydrophilic liquid phase, and an internal and lipophilic liquid phase, in order to form a capsule constituted of a core comprising the internal and lipophilic phase, and a shell comprising the external and hydrophilic phase,
- immersing into an aqueous solution containing a curing agent comprising calcium ions,
wherein the external liquid phase includes the gelling agent comprising gellan gum alone or in combination with another gelling agent, the filler, and the bivalent metal sequestering agent.

12. The process for manufacturing a seamless breakable capsule according to claim 11, comprising:
- co-extruding an external and hydrophilic liquid phase, and an internal and lipophilic liquid phase, in order to form a capsule constituted of a core comprising the internal and lipophilic phase and a shell comprising the external and hydrophilic phase,
- optionally solidifying and/or gelling the surface of the shell by keeping the capsule under cold conditions,
- optionally washing the so-obtained capsule with an organic solvent,
- immersing into an aqueous solution containing a curing agent comprising calcium ions,
- optionally drying the capsule.

13. The process for manufacturing a seamless breakable capsule according to anyone of claims 11 to 12, wherein the aqueous solution containing a curing agent is a calcium chloride solution, which pH is preferably, of 3 to 4.

14. The process for manufacturing a seamless breakable capsule according to anyone of claims 11 to 13, wherein the gelling agent is a combination of gellan and at least one other gelling agent selected from the group consisting of gelatin and hydrocolloids such as agar, carragheenan, pectins, xanthan gum, cellulose gum, alginate, dextran, curdlan, welan gum, rhamsan gum or modified starches, and mixtures thereof.

15. The process for manufacturing a seamless breakable capsule according to anyone of claims 11 to 14, wherein the filler is a starch derivative such as dextrin, maltodextrin, cyclodextrin, a cellulose derivative such as HPMC, HPC, MC and mixtures thereof.

16. The process for manufacturing a seamless breakable capsule according to anyone of claims 11 to 15, wherein the sequestering agent is a metal salt, preferably selected from the group comprising sodium carbonate, trisodium citrate, trisodium phosphate, tetrasodium pyrophosphate, sodium hexametaphosphate and mixtures thereof.

17. The process for manufacturing a seamless breakable capsule according to anyone of claims 11 to 16, wherein the external hydrophilic liquid phase further comprises a plasticizer, preferably selected from the group consisting of glycerol, sorbitol, maltitol, triacetine or PEG type, or another polyol with plasticizing properties, and mixtures thereof.

18. Slurry containing breakable capsules according to anyone of claims 1 to 10, in suspension in a gel formed with a gel forming agent such CMC, xanthan gum, or Carbopol, and optionally comprising preservatives and stabilizers.

19. Food product including breakable capsules according to anyone of claims 1 to 10.

20. Oral care product including breakable capsules according to anyone of claims 1 to 10.

21. Pharmaceutical product including breakable capsules according to anyone of claims 1 to 10.

22. Fragrance including breakable capsules according to anyone of claims 1 to 10.

## Patentansprüche

1. Nahtlose brechbare Kapsel, die einen Kern und eine Hülle umfasst, wobei die Hülle ein Geliermittel, das Gellangummi allein oder in Kombination mit einem anderen Geliermittel umfasst, beinhaltet,
**dadurch gekennzeichnet, dass** die Hülle weiterhin einen Füllstoff und einen Komplexbildner für ein zweiwertiges Metall ausgewählt aus der Gruppe Trinatriumcitrat, Trinatriumphosphat, Tetranatriumpyrophosphat, Natriumhexametaphosphat und Mischungen davon beinhaltet, wobei die Menge des Füllstoffs in der Hülle 50 bis 95 Gew.-% des gesamten Trockengewichts der Hülle ausmacht und die Menge des Komplexbildners maximal 2 Gew.-% des Gesamttrockengewichts der Hülle ausmacht.

2. Nahtlose brechbare Kapsel nach Anspruch 1, wobei der größere Durchmesser der Kapsel 0,5 mm bis 8 mm beträgt.

3. Nahtlose brechbare Kapsel nach Anspruch 1 oder 2, wobei es sich bei dem Geliermittel um eine Kombination aus Gellan und einem Geliermittel ausgewählt aus der Gruppe Gelatine, Agar-Agar, Carrageen, Pektine, Xanthangummi, Cellulosegummi, Alginat, Dextran, Curdlan, Welangummi, Rhamsangummi oder modifizierte Stärken handelt.

4. Nahtlose brechbare Kapsel nach Anspruch 1, wobei es sich bei dem Geliermittel ausschließlich um Gellangummi handelt.

5. Nahtlose brechbare Kapsel nach Anspruch 1, wobei bei Verwendung in Kombination mit mindestens einem weiteren Geliermittel das Gewichtsverhältnis zwischen Gellangummi und dem anderen Geliermittel bzw. den anderen Geliermitteln 80/20 bis 20/80, vorzugsweise 75/25 bis 25/75, noch stärker bevorzugt 60/40 bis 50/50, beträgt.

6. Nahtlose brechbare Kapsel nach einem der Ansprüche 1 bis 5, wobei es sich bei dem Füllstoff um ein Stärkederivat wie Dextrin, Maltodextrin, Cyclodextrin und/oder um ein Cellulosederivat wie Hydroxypropylmethylcellulose (HPMC), Hydroxypropylcellulose (HPC), Methylcellulose (MC), Polyvinylalkohole, Polyole oder Mischungen davon handelt.

7. Nahtlose brechbare Kapsel nach einem der Ansprüche 1 bis 6, wobei die Hülle weiterhin ein Säuresalz ausgewählt aus der Gruppe umfassend zum Beispiel Citrat, Glucuronat, Adipat, Fumarat, Gluconat und Salz des Glucono-Delta-Lactons und Mischungen davon umfasst.

8. Nahtlose brechbare Kapsel nach einem der Ansprüche 1 bis 7, die eine Druckfestigkeit von 0,01 bis 5 kp aufweist.

9. Nahtlose brechbare Kapsel nach einem der Ansprüche 1 bis 8, wobei die Menge an Weichmacher 0,1 bis 30 Gew.-%, vorzugsweise 2 bis 15 Gew.-% und noch stärker bevorzugt 3 bis 10 Gew.-% des Gesamttrockengewichts der Hülle ausmacht.

10. Nahtlose brechbare Kapsel nach einem der Ansprüche 1 bis 9, die eine Druckfestigkeit von 0,01 bis 5 kp aufweist.

11. Verfahren zur Herstellung einer nahtlosen brechbaren Kapsel nach einem der Ansprüche 1 bis 10, das Folgendes umfasst:
- Coextrudieren einer äußeren hydrophilen flüssigen Phase und einer inneren lipophilen flüssigen Phase, wodurch man eine Kapsel erhält, die aus einem Kern, der die innere lipophile Phase umfasst, und einer Hülle, die die äußere hydrophile Phase umfasst, besteht,
- Eintauchen in eine wässrige Lösung, die ein calciumionenhaltiges Aushärtungsmittel enthält,
wobei die äußere flüssige Phase das Geliermittel, das Gellangummi allein oder in Kombination mit einem anderen Geliermittel umfasst, den Füllstoff und den Komplexbildner für das zweiwertige Metall beinhaltet.

12. Verfahren zur Herstellung einer nahtlosen brechbaren Kapsel nach Anspruch 11, das Folgendes umfasst:
- Coextrudieren einer äußeren hydrophilen flüssigen Phase und einer inneren lipophilen flüssigen Phase, wodurch man eine Kapsel erhält, die aus einem Kern, der die innere lipophile Phase umfasst, und einer Hülle, die die äußere hydrophile Phase umfasst, besteht,
- gegebenenfalls Verfestigen und/oder Gelieren der Oberfläche der Hülle **dadurch**, dass die Kapsel unter kalten Bedingungen gehalten wird,
- gegebenenfalls Waschen der so erhaltenen Kapsel mit einem organischen Lösungsmittel,
- Eintauchen in eine wässrige Lösung, die ein calciumionenhaltiges Aushärtungsmittel enthält,
- gegebenenfalls Trocknen der Kapsel.

13. Verfahren zur Herstellung einer nahtlosen brechbaren Kapsel nach einem der Ansprüche 11 bis 12, wobei es sich bei der wässrigen Lösung, die ein Aushärtungsmittel enthält, um eine Calciumchloridlösung mit einem pH-Wert von vorzugsweise 3 bis 4 handelt.

14. Verfahren zur Herstellung einer nahtlosen brechbaren Kapsel nach einem der Ansprüche 11 bis 13, wobei es sich bei dem Geliermittel um eine Kombination von Gellan und mindestens einem weiteren Geliermittel ausgewählt aus der Gruppe Gelatine und Hydrocolloide wie Agar-Agar, Carrageen, Pektine, Xanthangummi, Cellulosegummi, Alginat, Dextran, Curdlan, Welangummi, Rhamsangummi oder modifizierte Stärken und Mischungen davon handelt.

15. Verfahren zur Herstellung einer nahtlosen brechbaren Kapsel nach einem der Ansprüche 11 bis 14, wobei es sich bei dem Füllstoff um ein Stärkederivat wie Dextrin, Maltodextrin, Cyclodextrin, ein Cellulosederivat wie HPMC, HPC, MC und Mischungen davon handelt.

16. Verfahren zur Herstellung einer nahtlosen brechbaren Kapsel nach einem der Ansprüche 11 bis 15, wobei es sich bei dem Komplexbildner um ein Metallsalz, vorzugsweise ausgewählt aus der Gruppe umfassend Natriumcarbonat, Trinatriumcitrat, Trinatriumphosphat, Tetranatriumpyrophosphat, Natriumhexametaphosphat und Mischungen davon handelt.

17. Verfahren zur Herstellung einer nahtlosen brechbaren Kapsel nach einem der Ansprüche 11 bis 16, **dadurch gekennzeichnet, dass** die äußere hydrophile flüssige Phase weiterhin einen Weichmacher, vorzugsweise aus der Reihe Glycerin, Sorbit, Maltit, Triacetin oder PEG-Typ, oder ein anderes Polyol mit Weichmachereigenschaften, sowie Mischungen davon, enthält.

18. Aufschlämmung, die brechbare Kapseln nach einem der Ansprüche 1 bis 10 in Suspension in einem mit einem Gelbildner wie CMC, Xanthangummi oder Carbopol gebildeten Gel enthält und gegebenenfalls Konservierungsmittel und Stabilisatoren umfasst.

19. Nahrungsmittelprodukt, das brechbare Kapseln nach einem der Ansprüche 1 bis 10 beinhaltet.

20. Mundpflegeprodukt, das brechbare Kapseln nach einem der Ansprüche 1 bis 10 enthält.

21. Pharmazeutikum, das brechbare Kapseln nach einem der Ansprüche 1 bis 10 enthält.

22. Duftstoff, der brechbare Kapseln nach einem der Ansprüche 1 bis 10 enthält.

## Revendications

1. Capsule cassable sans soudure comprenant une partie centrale et une coque, dans laquelle la coque comprend un agent gélifiant comprenant de la gomme de gellane seule ou en combinaison avec un autre agent gélifiant,
**caractérisée en ce que** la coque comprend en outre une charge et un agent séquestrant de métal divalent choisi dans le groupe constitué par le citrate trisodique, le phosphate trisodique, le pyrophosphate tétrasodique, l'hexamétaphosphate de sodium et leurs mélanges, la quantité de charge dans la coque étant de 50 % à 95 % en poids par rapport au poids sec total de la coque, la quantité d'agent séquestrant étant d'au plus 2 % en poids du poids sec total de la coque.

2. Capsule cassable sans soudure selon la revendication 1, dans laquelle le plus grand diamètre de la capsule est de 0,5 mm à 8 mm.

3. Capsule cassable sans soudure selon la revendication 1 ou 2, dans laquelle l'agent gélifiant est une combinaison de gellane et d'un agent gélifiant choisi dans le groupe constitué par la gélatine, l'agar, le carraghénane, les pectines, la gomme de xanthane, la gomme de cellulose, l'alginate, le dextrane, le curdlane, la gomme de welane, la gomme de rhamsane ou les amidons modifiés.

4. Capsule cassable sans soudure selon la revendication 1, dans laquelle l'agent gélifiant est la gomme de gellane seule.

5. Capsule cassable sans soudure selon la revendication 1, dans laquelle, quand il est utilisé en combinaison avec au moins un autre agent gélifiant, le rapport en poids entre la gomme de gellane et l'autre ou les autres agent(s) gélifiant(s) est de 80/20 à 20/80, de préférence de 75/25 à 25/75, et de manière davantage préférée de 60/40 à 50/50.

6. Capsule cassable sans soudure selon l'une quelconque des revendications 1 à 5, dans laquelle la charge est un dérivé d'amidon, comme la dextrine, la maltodextrine, une cyclodextrine et/ou un dérivé de cellulose, comme l'hydroxypropylméthyl-cellulose (HPMC), l'hydroxypropylcellulose (HPC), la méthylcellulose (MC), les alcools polyvinyliques, les polyols ou leurs mélanges.

7. Capsule cassable sans soudure selon l'une quelconque des revendications 1 à 6, dans laquelle la coque comprend en outre un sel d'acide choisi dans le groupe comprenant un citrate, un glucuronate, un adipate, un fumarate, un gluconate et un sel de glucono-delta-lactone, et leurs mélanges.

8. Capsule cassable sans soudure selon l'une quelconque des revendications 1 à 7, présentant une résistance à l'écrasement allant de 0,01 à 5 kp.

9. Capsule cassable sans soudure selon l'une quelconque des revendications 1 à 8, dans laquelle la quantité de plastifiant est de 0,1 % à 30 % en poids, de préférence de 2 % à 15 % en poids et de manière davantage préférée de 3 % à 10 % en poids du poids sec total de la coque.

10. Capsule cassable sans soudure selon l'une quelconque des revendications 1 à 9, présentant une résistance à l'écrasement allant de 0,01 à 5 kp.

11. Procédé de fabrication d'une capsule cassable sans soudure selon l'une quelconque des revendications 1 à 10, comprenant
- la co-extrusion d'une phase liquide externe et hydrophile et d'une phase liquide interne et lipophile afin de former une capsule composée d'une partie centrale comprenant la phase interne et lipophile et une coque comprenant la phase externe et hydrophile,
- l'immersion dans une solution aqueuse contenant un agent de durcissement comprenant des ions de calcium,
dans lequel la phase liquide externe comprend l'agent gélifiant comprenant la gomme de gellane seule ou en combinaison avec un autre agent gélifiant, la charge et l'agent séquestrant de métal divalent.

12. Procédé de fabrication d'une capsule cassable sans soudure selon la revendication 11, comprenant :
- la co-extrusion d'une phase liquide externe et hydrophile et d'une phase liquide interne et lipophile afin de former une capsule composée d'une partie centrale comprenant la phase interne et lipophile et une coque comprenant la phase externe et hydrophile,
- facultativement la solidification et/ou la gélification de la surface de la coque en maintenant la capsule dans des conditions froides,
- facultativement le lavage de la capsule ainsi obtenue avec un solvant organique,
- l'immersion dans une solution aqueuse contenant un agent de durcissement comprenant des ions de calcium,
- facultativement le séchage de la capsule.

13. Procédé de fabrication d'une capsule cassable sans soudure selon l'une quelconque des revendications 11 à 12, dans lequel la solution aqueuse contenant un agent de durcissement est une solution de chlorure de calcium, dont le pH va de préférence de 3 à 4.

14. Procédé de fabrication d'une capsule cassable sans soudure selon l'une quelconque des revendications 11 à 13, dans lequel l'agent gélifiant est une combinaison de gellane et d'au moins un autre agent gélifiant choisi dans le groupe constitué par la gélatine et des hydrocolloïdes tels que l'agar, le carraghénane, les pectines, la gomme de xanthane, la gomme de cellulose, l'alginate, le dextrane, le curdlane, la gomme de welane, la gomme de rhamsane ou les amidons modifiés, et leurs mélanges.

15. Procédé de fabrication d'une capsule cassable sans soudure selon l'une quelconque des revendications 11 à 14, dans lequel la charge est un dérivé d'amidon, comme la dextrine, la maltodextrine, une cyclodextrine, un dérivé de cellulose, comme l'HPMC, l'HPC, la MC et leurs mélanges.

16. Procédé de fabrication d'une capsule cassable sans soudure selon l'une quelconque des revendications 11 à 15, dans lequel l'agent séquestrant est un sel métallique, choisi de préférence dans le groupe comprenant le carbonate de sodium, le citrate trisodique, le phosphate trisodique, le pyrophosphate tétrasodique, l'hexamétaphosphate de sodium et leurs mélanges.

17. Procédé de fabrication d'une capsule cassable sans soudure selon l'une quelconque des revendications 11 à 16, dans lequel la phase liquide hydrophile externe comprend en outre un plastifiant, choisi de préférence dans le groupe constitué par le glycérol, le sorbitol, le maltitol, la triacétine ou un type de PEG, ou un autre polyol ayant des propriétés plastifiantes, et leurs mélanges.

18. Pâte contenant des capsules cassables selon l'une quelconque des revendications 1 à 10, en suspension dans un gel formé avec un agent formant un gel, comme le CMC, la gomme de xanthane ou le Carbopol, et comprenant facultativement des conservateurs et des stabilisants.

19. Produit alimentaire comprenant des capsules cassables selon l'une quelconque des revendications 1 à 10.

20. Produit de soin buccal comprenant des capsules cassables selon l'une quelconque des revendications 1 à 10

21. Produit pharmaceutique comprenant des capsules cassables selon l'une quelconque des revendications 1 à 10.

22. Fragrance comprenant des capsules cassables selon l'une quelconque des revendications 1 à 10.
